(19) Europäisches Patentamt
European Patent Office
Office européen des brevets

(11) EP 0 946 565 B1

(12) **EUROPEAN PATENT SPECIFICATION**

(45) Date of publication and mention of the grant of the patent:
**15.10.2003 Bulletin 2003/42**

(21) Application number: **97947981.3**

(22) Date of filing: **19.12.1997**

(51) Int Cl.[7]: **C07D 493/10**, B01D 11/02 // (C07D493/10, 307:00, 307:00)

(86) International application number:
**PCT/KZ97/00006**

(87) International publication number:
**WO 98/028303 (02.07.1998 Gazette 1998/26)**

(54) **METHOD AND DEVICE FOR PRODUCTION OF LYOPHILIZED HYDROCHLORIDE-1SS, 10SS-EPOXY-13-DIMETHYLAMINO-GUAIA-3(4)-EN-6,12-OLIDE**

VERFAHREN UND GERÄT ZUR HERSTELLUNG VON 1SS, 10SS-EPOXY-13-DIMETHYLAMINO-GUAIA-3(4)-EN-6,12-OLID-HYDROCHLORID

PROCEDE ET DISPOSITIF DE PRODUCTION D'HYDROCHLORURE-1SS, 10SS-EPOXY-13-DIMETHYLAMINO-GUAIA-3(4)-EN-6,12-OLIDE LYOPHILISE

(84) Designated Contracting States:
**AT CH DE FR GB IT LI NL SE**

(30) Priority: **20.12.1996 KZ 969951**
**23.04.1997 KZ 973971**

(43) Date of publication of application:
**06.10.1999 Bulletin 1999/40**

(73) Proprietor: **Tovarischestvo S Ogranichennoi Otvetstvennostju "Tabifarm"**
**Karagandan, 470032 (KZ)**

(72) Inventor: **ADEKENOV, Sergazy Mynzhasarovich**
**Karaganda, 470032 (KZ)**

(74) Representative: **von Füner, Nicolai, Dr. et al**
**v. Füner Ebbinghaus Finck Hano**
**Patentanwälte**
**Postfach 95 01 60**
**81517 München (DE)**

(56) References cited:

- **DATABASE WPI Week 9201 Derwent Publications Ltd., London, GB; AN 92-006101 XP002061054 & SU 1 627 208 A (CANNING VEGETABLE) cited in the application**
- **J. MARCH: "Advanced Organic Chemistry - Reactions, Mechanisms, and Structure. Third Edition," 1985 , JOHN WILEY & SONS , NEW YORK US XP002059381 see page 689 - page 691**
- **CHEMICAL ABSTRACTS, vol. 126, no. 20, 19 May 1997 Columbus, Ohio, US; abstract no. 263418v, S.M. ADEKENOV ET AL.: "Natural antifeedants and repellents for protection of agricultural commodities" page 608; column 2; XP002059382 & VESTN. S-KH. NAUKI KAZ., vol. 8, 1996, pages 56-57,**
- **CHEMICAL ABSTRACTS, vol. 98, no. 11, 14 March 1983 Columbus, Ohio, US; abstract no. 86299q, S.M. ADEKENOV ET AL.: "Arglabin - a new sesquiterpene lactone from Artemisia glabella" page 298; column 1; XP002059383 & KHIM. PRIR. SOEDIN., vol. 5, 1982, pages 655-656, XP002061053**

EP 0 946 565 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to pharmaceutical industry, to the sesquiterpene lactones and, particularly, to the method of production of lyophilized hydrochloride 1β, 10β - epoxy - 13 - dimethylamino - guaia - 3(4) - en - 6.12 - olide of the following formula

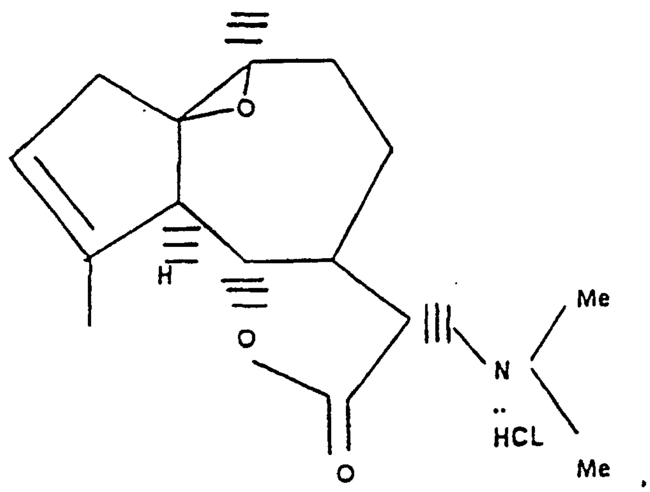

(I)

which has antitumour activity and can be used to develop new preparations for chemotherapy of malignant tumours.

According to S. M. Adekenov et al., *Khim. Prir. Soedin.* (5) (1982) 655 - 656, from the overground part of Artemisia glabella Kar. et Kir. (smooth wormwood), picked in August, 1978 (Kent Mountains, Karagandiskaya oblast, former Kazakh SSR), by means of water extraction, with subsequent extraction by chloroform, there has been obtained a number of substances. Using column chromatography of the resin with silica gel of benzene fractions, a crystalline substance of the formula $C_{15}H_{18}O_3$ has been separated, with a melting point of 100-102 °C (hexane), $[\alpha]^{20}_{D}$+45,6 °C, which has been shown to be a novel sesquiterpene lactone, and has been named arglabin.

S.M. Adekenov et al., *Vestn. S-kh. Nauki Kaz.* (8) (1996) 56 - 67 discloses aminoarglabin hydrochloride.

The method of production of arglabin lyophilised having antitumour activity from the natural material - flower baskets and leaves of endemic plant *Artemisia glabella Kar. et Kir.* was described in preliminary patent of the Republic of Kazakstan, N 5277, cl. CO7D 307/93, A61 K, 31/365, 1997).

According to this method initially dry grinded natural material is treated by a solvent in the extractor, then extracted substances (resin) are purified from worthless products through treatment by 60% aqueous solution of ethyl alcohol. The resin produced at the stage of purification is divided into separate components by the method of column chromatography on silicagel. The said column is eluted with benzene and the fractions containing arglabin are separated. Benzene is distilled and technical arglabin is produced which is further subjected to crystallisation, amination and hydrochloration. In this method amination is canied out by dimethylamine with production of a solution containing arglabin. Then the alcohol solution of aminoarglabin is barbotaged by chlorine hydride and the target product - aminoarglabin hydrochloride is produced. Amination and hydrochloration reactions are controlled by the methods of thin layer chromotography. Technical hydrochloride of aminoarglabin is purified by crystallization with subsequent drying and lyophilization.

Disadvantages of the above method are its rather low yield of target products in the amination and hydrochloration reactions.

Different devices are used for extraction, for example, the device consisting of extractor with a jacket in the extraction zone, evaporator, condenser and heater (the USSR author's certificate N 1117071, cl. BOID 11/02, 1984).

The USSR author's certificate N 670310, cl. BO1D 11/02, 1979 describes the device for gas - liquid extraction consisting of evaporator, condenser and extractor, where the extracted mixture is heated by the heat produced at condensation of extractant vapours transported from evaporator, said heat is transmitted through the wall of the extractor.

The disadvantage of all these devices is periodicity of their operation what cannot provide high productivity. These devices also do not provide extraction of the extractant from waste material what results in great losses of the extractant. In the USSR author's certificate N 1117071 reacting mixture is heated by an additional power supply what increases power consumption in the process of extract production.

The USSR author's certificate N 1426611, cl. BOID 11/00, 1988 describes the device for extraction in the solid -liquid system consisting of the body, extraction chamber with changeable container, evaporator and condenser which enables to distill the extractant from the solid phase by a warm inert gas at the final stage of extraction. This device also does not provide continuous operation and, respectively, has low productivity. Besides, when volatile extractant are distilled by a warm gas while condenser is cooled by water, the whole extractant can be taken away with the gas without its condensation in the condenser. For example, according to our calculations, to distill chloroform by a gas with initial temperature 70°C and to cool it in the condenser to 20°C, $5m^3$ of gas are required for evaporation of 1 kg of chloroform and for its condensation partial pressure of chloroform in the gas must be not lower than the saturation partial pressure at 20°C what corresponds to the content of 1kg of chloroform in $1m^3$ of gas. Thus, partial pressure of chloroform in the gas in the above example is 5 times lower than that required for condensation, i.e. no condensation is possible in the condenser and all the chloroform will leave the condenser together with the gas.

There are also some special devices for solvent removal from the waste material, for example, the method and device for removal of the solvent from solid substances, consisting of a capacity for waste material contact with heat-carrying agent, separators for extractant and heat-carrying agent separation, condensers for extractant vapours. (Patent EP 00356338, cl. BOID 11/00, 1990).

Such devices are complicated and energy consuming, besides, when waste material is reloaded from the extraction device to other devices, it is impossible to prevent extractant losses or it is necessary to use complex hermetic devices for reloading.

The closest to the claimed device is a section blade extractor of dry natural matetial of continuous operation consisting of cylindric body divided by partitions with openings into not less than three sections, separation and humidity chamber, shaft mounted on the same axis with the body with reloading ladles, mixing blades and loader are attached to said shaft (the USSR author's certificate N 1627208, cl. BOID 11/02, 1991).

This design provides increase in productivity as compared with the above devices but this increase is not sufficient. The disadvantage of this design is also high extractant consumption as this device does not enable to extract the extractant from waste material what results in extractant losses. The usage of other known devices to extract the extractant from waste material considerably increases the cost of the installation and requires additional devices providing reloading and transportation of wet waste raw material without extractant emission to the environment.

The purpose of the present invention is to develop a method and device which could provide high yield of target product, complete extraction of the extract from raw material, increase in productivity of the extraction device , reduction of power supply, minimization of extractant losses, exclusion of extractant emission in the environment and, thus, guarantee of environmentally safe production.

According to the invention these tasks are solved by claims 1 and 3 of the Claims. Preferred embodiments of the invention are given in dependent claims.

The method of production of lyophilized hydrochloride 1β, 10β - epoxy - 13 - dimethylamino - guaia - 3(4) - en - 6,12 - olide - antitumour preparation from endemic plant *Artemisia glabella Kar.et Kir*. comprises resin extraction from raw material, its purification from worthless side substances, separation of said resin into separate components by column chromatography with production of technical Arglabin which is further subjected to recrystallization, amination and hydrochloration with production of aminoarglabin hydrochloride and its further recrystallization, drying, lyophilization and identification, wherein amination is carried out by solution of crystalline arglabin in alcohol with addition of dimethylamine in said alcohol solution till pH of the solution reaches 12.3 - 12.4, and wherein to extract aminoarglabin hydrochloride from said solution containing aminoarglabin alcohol is distilled and chloroform is filled in, the remaining water is removed, said solution is filtered, said chloroform is evaporated and alcohol added, then the solution is barbotaged by chlorine hydride to 5.0 - 5.5 pH solution, alcohol is evaporated and ethylathetate added.

It is preferable to carry out identification of arglabin by IR-spectroscopy.

In the claimed method stages of amination and hydrochloration under given operation parameters of pH-solutions provide high yield of 13-dimethylaminoarglabin and 13-dimethylaminoarglabin hydrochloride. In its turn, it provides high output of target product.

The invention also includes the device to implement this method. Technical result of the present invention is achieved by the device for arglabin extraction from natural raw material consisting of horizontal extractor including cylindrical body divided by partitions with openings into not less than three sections, separation and humidity chambers, shaft mounted on the same axis with the body with reloading ladles, blade mixers and the loader attached to it, the said extractor is provided with additional section with heated walls to remove extract from waste material and dry waste material collector, the walls of contact sections are water-cooled, cavities of cooling jackets of upper parts of the walls of said section being in contact with vapour-gas medium and lower parts of the walls being in contact with extraction mixture are not connected with each other, the system of gas circulation is connected to the extractor cavity, the said system consists of branch pipe for gas supply connected to dry waste material collector, branch pipe for gas removal connected to the cavity of the contact section of the extractor, low temperature condenser and ventilator, lead screw mounted in the separation chamber is connected to said shaft of the extractor and the partition between the separation

and the humidity chambers is supplied with additional opening in its upper part.

According to one embodiment of the invention, the cylindrical body of the extractor is mounted with an inclination.

The other embodiment of the invention is that the branch pipe for the extract removal from the separation chamber of the extractor is connected to the filtering centrifuge connected in series with evaporator and condenser.

The advantage of the invention is that as compared with the known devices the present design provides heating of extracted mixture and keeping of its temperature at a required level due to condensation of extractant vapour inside the extractor and usage of condensation heat for heating of extracted mixture what results in increase in the productivity of extraction and reduction in extractant consumption and power supply.

The device contains preferred additional section with heated walls designed to remove extractant from waste material what enables to reduce extractant losses.

The device is supplied with the system for gas circulation which removes the air entering the extractor in the pores of raw material and, thus, prevents emission of extractant vapours into atmosphere and excludes extractant losses, as well as removes air or other gas supplied to replace extractant vapour in dry waste material pores before its unloading what also reduces extractant losses.

The device provides continuous removal of not only precipitated but also of suspended particles from the separation chamber after purification of the extract before its withdrawal from the extractor.

The preferred technology provides environmentally safe operation of the device without extractant emission in the environment and deep gases precipitation from the extractor. before their emission to the atmosphere.

The present extractor provides continuous process of multi-stage extraction of final product by liquid extractant with continuous mixing of extraction mixture at optimal extraction temperature and counterflow of raw material and extractant that ensures the most complete extraction of the final product out of raw material and maximal productivity without extractant losses. Below the invention is explained in details on the examples of its implementation.

Fig. 1 shows the extractor, its longitudinal section.Fig.2 shows the scheme of the device for the extraction of arglabin from raw material.

The extractor consists of a cylindric inclined body 1 divided by vertical partitions 2 and 3 into sections. Partition 4 divides the first section into separation 5 and humidity 6 chambers. Along the axis of the body, the rotating shaft 7 is mounted, reloading ladles 8 and blade mixers 9 are attached to it. In separation chamber 5 (sedimentator) peripheral lead screw with a small gap to the body is placed, its internal edge in the upper point of the extractor is dipped into liquid. In the upper part of the partition 4 the opening 1I is made, its lower edge is located at the liquid level. There is also the opening 12 in the lower part of partition 4. The loading device 13 with feeder and bin for raw materials is mounted on the humidity chamber 6 in the first section of the extractor. There are two openings 7 in the partitions 2, the upper opening 14 is intended for reloading of raw material to the next section and the lower opening 15 closed by mesh is designed for extractant transfer.

There can be one or several contact sections 16 in the extractor. In the last section with respect to raw material motion, contact section 17, there is no opening in the partition 3 and said partition 3 is hermetically sealed in the part which is lower than the level of extraction mixture.

On the body 1 of the extractor near contact sections 16, 17, water jackets 18 and 19 are made. Water jacket 18 is placed higher than the extracted mixture level in the extractor and water jacket 19 is placed lower than that level. The cavities of both jackets are not connected with each other and have separate branch pipes for supply and discharge of cooling water.

Next to the last contact section 17 along the raw material motion, there is an additional section 20 (dryer) designed to remove extractant from the waste raw material. On the body of section 20, predominantly in its lower part being in contact with the waste material, the heater 21, for example, an electric heater, is mounted. In section 20 the collector of dry waste material 22 is placed, it is supplied with a hinged out window 23 with a grid, the unloading port of collector 22 is closed by easily removed lid 24. The canal 25 is designed to supply air or inert gas to ventilate waste material in the collector 22. The gas is heated, for example, by heater 21 or a special gas heater. In the upper point of the body I near the contact section 16 there is the branch pipe 26 for gas removal from the extractor, the said branch pipe is connected with low temperature condenser 27 cooled by the coolant supplied by the refrigerator. The ventilator 28 is designed to draw off the gases.

The extractor operates as follows:

Dry grinded natural raw material (flower baskets and leaves of *Artemisia glabella Kar.et Kir.*) is supplied through the feeder 16 into the humidity chamber 6, where it is mixed with extractant by mixers 9. The raw material impregnated with extractant is reloaded by reloading ladles 8 through the port 14 in partitions 2 into contact section 16 where it is continuously mixed with extractant by mixers 9 and moved to the next section. Further the raw material is transported by reloading ladles 8 to the section 17 into which the newly made extractant is supplied from the opposite side. In the section 17 the raw material is washed by a newly made solvent, released from the residue of the target product and by reloading ladles is transported to the section 20 where the extractant is evaporated from waste material

during its contact with hot walls. Mixers 9 are used to mix the waste material and to achieve uniform drying and to transport it to waste material collector 22 where the extractant vapour is displaced from the pores of waste material by heated air or inert gas supplied under the grid 23. As the collector 22 is filled, the dry ventilated waste material is discharged, to do this the grid 23 is lowered and the lid 21 is opened. The extractant vapour flows through the upper opening in partition 4 from the drying section 20 to the section 17 where the most part of said vapour is condensed on the surfaces cooled by water passing through the upper water jacket 18 as well as on the surface of extracted mixture heating it. Condensed extractant flows down to the extracted extractant mixture. The residue of extractant vapour together with the air flows through the upper opening in the partition into the contact section 16 where they are condensed to partial saturation pressure corresponding to the temperature of the vapour - gas mixture drawn off from the extractor. The temperature of the extracted mixture is regulated by the amount of cooling water supplied to the lower water jacket 19. Gases from the extractor containing a small amount of extractant vapours are transported through the branch pipe 26 to the low temperature condenser 27 cooled by the coolant from a refrigerator machine, in the said condensor extractant vapours are almost completely condensed from gases. The condensate from the condenser 27 is returned to the extractor. The gas cleared from extractant vapours is emitted through the discharge ventilator 28 to the atmosphere. The air entering the extractor with the raw material in its pores is drawn off in the same way through the branch pipe 26.

The newly made extractant enters section 17 near the unloader of waste material, the condensed extractant from the drying section 20 runs down to the same place. The extractantin the reactor moves from section to section through the lower openings 15 in the partitions in the opposite direction to the raw material flow. Grids on these openings prevent opposite motion of raw material. From the humidity chamber 6 the extractant through the lower opening in the partition 4 enters the separation chamber 5 where it is purified, heavy particles go down and light particles rise up and are removed by the peripheral lead screw from the separation chamber to the humidity chamber, suspended particles are removed through the upper opening 11 and precipitated particles are removed through the lower opening 12. Purified extract (extractant saturated with extracted substances) is transported from the near - axis zone of the extractor for the further processing as it is shown in fig.2.

According to the scheme given in fig.2 the extraction device consists of continuously operating counterflow section extractor 1 with additional section 2 with heated walls for extractant removal from waste material with common drive 3. The loader consisting of intermediate bunker 4, feeder 5 with drive 6 and canal 7 is mounted on the extractor. The changed capacity 8 with raw material is placed on the intermediate bunker 4, a standard craft-sack is used as changed capacity. The pump 9 is intended to feed the extract from the extractor 1 to the filtering centrifuge 10 placed on the extractor where the extract is cleared from suspended particles. Particles precipitated from the extract in the centrifuge are discharged from the centrifuge to the last stage of the extractor. Then the extract is feeded to the film-type evaporator 11 with built - in steam generator for condensation. The condensed extract is periodically poured out from the condenser 11 to the changeable capacities 12. The evaporator 11 is connected with the water-cooled condenser 13 from which the distilled extractant is transported to the buffer tank 19. Pure extractantis transported from the tank 19 to the last stage of the extractor and moved to the first stage in the direction opposite to that of the raw material flow, extracting target substances from it. The tank 19 is equipped with level meter, flow meter and regulator of extractant and flow.

The extractor 1 is equipped with the system for gas removal which consists of condenser 14 cooled by water, freezer 15 which simultaneously serves as an evaporator for freon refrigerator machine, in the capacity of which the freezer of a household fridge is used, and ventillator 17. Gases from the extractor are transported to the condenser where the excess of extractant vapours, not having condensed on the walls of the extractor, is condensed. Further, the gases enter the freezer 15, the temperature of its walls is kept at -15-20°C by the refrigerator machine 16, and the extractant vapours remained in gases condense on its walls. Then gases practically completely cleared from extractant vapours are discharged by the ventilator 17 to the common ventilation system in the building.

The worked out and dried raw material is collected into easily removed changeable capacity 18 (a standard craff-sack).

Instruments for direction, control, regulation, signalling and protection of the device are mounted on the control panel 20. All units of the device operate under low pressure what excludes penetration of the vapour into the premises.

The device is mounted on its own frame and does not need special basement. Its operation is attended by 1-2 specially trained operators.

The design of the device enables without its dismounting to completely clean the device from the extractant without losses into the environment and almost completely (95-98%) from raw material when the device is prepared for repairings or change of raw material or extractant.

The design of the device enables to observe its operation and readjust it without unloading of raw material or extractant and without extractant emission to the environment.

During working day 0.539 kg of resin (the total amount of extracted substances) is produced what amounts to 7% with respect to dry raw material. The resin produced is further transported to stage II where it is purified from

side substances.

For this purpose the resin is treated by 1.078 liter of ethyl alcohol heated to 60°C, the resin is solved in the said alcohol under mixing. Then heated distilled water is poured in the volume ratio of ethyl alcohol to water equal to 2:1. The mixture is mixed carefully during 30 minutes and kept at room temperature without mixing during 24 hours. Side substances precipitate during this time. In a 24-hour period the sedimentation is mixed by the mixer and alcohol-water solution is filtered by vacuum. Alcohol as an azeothropic mixture with water containing 68-70% of alcohol is distilled from the obtained filtered substance under vacuum and at temperature of 50-52°C. The yield of resin amounts to 286g.

In stage III the resin produced in stage II is divided into individual components by column chromatography. Benzene is fed under pressure in the column and benzene fraction containing arglabin is separated. The presence of arglabin in the benzene fraction is determined by the method of thin layer chromatography on Silufol plates. After benzene distilling from fractions, there remains technical arglabin with an admixture of yellow oil amounting to 33.1g what equals to 11.7% of the processed resin.

Stage IY is a process of arglabin recrystallization. For this, technical arglabin is dissolved in hexane while heating on water bath at the ratio of product (kg) - solvent (1) equal to 1:10 and filtered under vacuum. During cooling to room temperature the crystals of arglabin start to isolate from the filtrate. After complete hexane removal, arglabin yield amounts to 21g (63%).

At stages Y and YI arglabin is aminated and hydrochlorated. To produce aminoarglabin, crystallic arglabin is dissolved in alcohol. Then dimethylamine is added to the alcohol solution till bringing pH to 12.3-12.4. Then alcohol is distilled from the solution containing aminoarglabin and chloroform is poured in. The main part of the water is separated by separating funnel. Traces of the remaining water are removed by one of the dryers ($MgSO_4$ or $Na_2SO_4$) till total transparency is achieved, the duration of treatment is about 12-14 hours. The solution is filtered, chloroform is evaporated and alcohol is added. Then said solution is barbotaged by chlorine hydride till pH reaches 5.0-5.5. Alcohol is evaporated from the solution and ethylacetate is added. The crystals of 13-dimethylaminoarglabin hydrochloride are precipitated. Its yield is 105%. The solution is evaporated till dry and technical aminoarglabin hydrochloride in the form of a powder is produced.

At stage YII aminoarglabin hydrochloride (technical product) is purified by fractional recrystallization. The product is dissolved in chloroform, then said chloroform is removed on rotatory evaporator. Under intensive stirring ethylacetate is added to the remained resin. The precipitated sedimentary of aminoarglabin hydrochloride is filtered through vacuum - pistol and used for the production of arglabin - lyophilized preparation. The yield of arglabin-lyophilized (target product) is 20g (95% at this stage).

At the last stage YIII the produced target product is dried over anhydrone under vacuum, dissolved with apyretic distilled water at ratio: 2g of dry subtauce per 100 ml of water. The produced solution is lyophilized by one of the following methods.

***Method 1.*** Produced aqueous solution is passed through cotton-gauze tampon in the tank with Millipor filter. The solution is filtered through sterile Millipor trimmed by filtering plates into a sterile glass vessel. Then the solution is pumped by means of vacuum from the glass vessel into a measuring burette from which it is poured into 2ml bottles and dried in lyophilizer KS-30. To do this the bottles are placed on trays. When the trays are filled with bottles, they are wrapped into a sterile sheet and placed on freezing counter for hardening at -40°C for not less than 24 hours. After this procedure drying is commenced. In 2 hours after the beginning of drying, heating of the shelves is turned on. The shelves are heated gradually up to +50±5°C. The product turns from negative to positive temperature at 12th-13th hour of drying. The final temperature of the product should not exceed +60°C.

Duration of drying is 24 hours. After the lyophilic drying, the bottles with preparation are immediately closed by the corks, covered by caps and rolled. Each bottle contains 0.04g (0.00004 kg) of preparation.

***Method 2.*** Aqueous solution is filtered through cotton-gauze tampon or 8 layers of gauze, poured into 2 ml bottles or ampules and lyophilized under the above conditions. The bottles with dry preparation are immediately closed by the corks, covered with caps and rolled. Ampules are soldered. Bottles or ampules with preparation are packed up in bicks or double-layered sacks and sterilized in autoclave during 20 minutes at the pressure of 1.2 Atm and temperature 120°C. Each bottle (ampula) contains 0.4g (0.00004 kg) of the preparation.

***Method 3.*** Prepared aqueous solution is filtered through cotton-gauze tampon or 8 gauze layers, poured by 200ml into flasks of volume of 500ml, closed by cotton-gauze tampons and tied up by oil-paper. The flasks with solution are sterilized in autoclave during 30 minutes at 1.2 Atm and the temperature of 120°C. The sterile solution is cooled to room temperature, sterily poured by 2ml into the bottles with the volume of 10ml and dried in the lyophilizer as stated above. After lyophilization, each bottle contains 0.04 (0.00004 kg) of preparation.

The yield of preparation is 17g what amounts to 88.2% at this stage and 0.22% with respect to air-dry natural material. Arglabin lyophylized preparation is a straw - white compound with a bitter taste.

According to the present invention identification of arglabin lyophilized is made by IR-spectroscopy. Spectrum of 1% of standard arglabin sample solution in chloroform is analysed by IR-spectrometer. The spectrum has characteristic absorption lines at 2800, 1775, 1650 $cm^{-1}$.

The 1775 $cm^{-1}$ line is characteristic for carbonyl group of γ-lactane and it does not overlap with other spectrum lines, therefore it is used to calculate the coefficient of absorption for the solution of a standard arglabin sample.

The coefficient of absorption is calculated by the formula:

$$a = \frac{A}{c_1 \cdot c_2 \cdot b}$$

where A is an optical density ;
**$c_1$** is a fraction of investigated compound in the sample; %
**$c_2$** is sample concentration in the analysed solution, %;
**b** is the thickness of cuvette, mm.

Content of arglabin in the standard sample is equal to 100 %. Concentration of the sample in the analysed solution is 1 %, cuvette thickness is 0.4 mm.

$$a = \frac{0.9852}{(1)\cdot(100)\cdot 0.4} = 0.02463$$

To make quantitative estimation, IR- spectrum of arglabin solution in chloroform with known concentration is measured. The band width of T % at 1775 cm $^{-1}$ is transformed into optical density and arglabin concentration is calculated by the formula:

$$C_2 = \frac{A}{a\cdot b\cdot c_1}$$

Percentage concentration of arglabin is detennined by spectrophotometer and it is not less than 99.9 %.

The control of the quality of preparation can also be made by the method of thin layer chromatography, qualitative reaction of preparation interaction with solution in sulphuric acid , determination of melting temperature, specific rotation.

Antitumour activity and toxicity of the substance and medicinal form of the present preparation of dimethylaminoarglabin hydrochloride was determined according to commonly used techniques.

Thus, the present method and device enable to produce highly-effective antitumour preparation of the natural origin - arglabin lyophilised possessing high antitumour and immuno-stabilizing activity and relatively low toxicity.

## Claims

1. A method of production of lyophilised hydrochloride 1β, 10β - epoxy-13-dimethyl amino - guaia - 3(4) - en - 6, 12-olide from endemic plant *Artemisia glabella Kar. et Kir*. including resin extraction from the initial product, its purification from worthless substances, said resin separation into separate components by the method of column chromatography with production of technical arglabin which is further subjected to recrystallisation, amination and hydrochloration with production of aminoarglabin hydrochloride and its further recrystallisation, drying, lyophilisation and identification in which:

   said amination is carried out by solving crystalline arglabin in alcohol with addition of dimethylamine to the said solution till pH of said solution reaches 12.3 - 12.4, at the previous stage to extract aminoarglabin hydrochloride containing aminoarglabin, alcohol is distilled and chloroform is filled in, the remaining water is removed, said solution is filtered, said chloroform is evaporated and alcohol added, the said solution is barbotaged by chlorine hydride till the pH of the solution reaches 5.0 - 5.5, said alcohol is evaporated and ethylacetate is added.

2. The method as claimed in claim 1, in which identification of arglabin lyophilised is made by IR-spectroscopy.

3. A device for use in the extraction of arglabin from natural material according to claim 1 consisting of horizontal extractor including cylindrical body divided by partitions with openings into not less than three sections, separation and humidity chamber, the shaft mounted on the same axis with said body, ladles and blade mixers attached to

said shaft, and the loader, in which

the said extractor is supplied with an additional section with heated walls to remove the extract from waste material and collector of dry waste material, the walls of contact sections are water-cooled, the cavities of cooling jackets in the upper parts of the walls, being in contact with vapour-gas medium, and in the lower parts of the walls, being in contact with the extracted mixture, are not open to each other,

said system of gas circulation is connected to said extractor cavity, said system for gas supply includes branch pipe connected to the cavity of contact section of the extractor, low temperature condenser and ventilator,

the lead screw placed in the separation chamber is connected to the shaft of the extractor,

the partition between the separation and the humidity chambers is made with additional opening in its upper part.

**4.** The device as claimed in claim 3, in which the cylindrical body of the extractors is inclined.

**5.** The device as claimed in claim 3 or claim 4, in which the branch pipe for the extract removal from the separation chamber of the extractor is connected to the filtration centrifuge which is connected with the evaporator and condensor in series.

## Patentansprüche

**1.** Verfahren zur Herstellung des lyophilisierten Hydrochlorids 1β,10β-Epoxy-13-dimethylamino-guaia-3(4)-en-6,12-olid aus der endemischen Pflanze Artemisia glabella Kar. et Kir., einschließlich der Harzextraktion aus dem Ausgangsprodukt, seiner Reinigung von wertlosen Substanzen, der Auftrennung des Harzes in einzelne Komponenten nach der Methode der Säulenchromatographie unter Erzeugung von technischem Arglabin, das dann weiter umkristallisiert, aminiert und hydrochloriert wird, wodurch Aminoarglabinhydrochlorid erzeugt wird, und seiner Umkristallisation, Trocknung, Lyophilisierung und Identifizierung,
bei dem die Aminierung durch Lösen von kristallinem Arglabin in Alkohol unter Zugabe von Dimethylamin zur Lösung, bis der pH der Lösung einen Wert von 12,3 bis 12,4 erreicht, auf der obigen Stufe zur Extraktion des Aminoarglabin enthaltenden Aminoarglabinhydrochlorids durchgeführt wird, der Alkohol abdestilliert wird und Chloroform eingefüllt wird, das restliche Wasser entfernt und die Lösung filtriert wird, das Chloroform abgedampft und Alkohol zugesetzt wird, wonach man durch die Lösung Chlorwasserstoff durchperlen lässt, bis der pH der Lösung einen Wert von 5,0 bis 5,5 erreicht, wonach der Alkohol abgedampft und Ethylacetat zugesetzt wird.

**2.** Verfahren nach Anspruch 1, bei dem die Identifizierung des lyophilisierten Arglabins durch IR-Spektroskopie erfolgt .

**3.** Vorrichtung zur Verwendung bei der Extraktion von Arglabin aus natürlichem Material gemäß Anspruch 1, die in einem horizontalen Extraktor besteht, der einen zylindrischen Körper, der durch Trennwände mit Öffnungen in mindestens drei Abschnitte gegliedert ist, eine Trenn- und Feuchtigkeitskammer, wobei die Welle auf derselben Achse wie der zylindrische Körper montiert ist, an der Welle montierte Pfannen, Schaufelmischer und die Beschikkungsvorrichtung umfasst,
wobei der Extraktor mit einem zusätzlichen Abschnitt mit erwärmten Wänden zur Entfernung des Extrakts aus dem Abfallmaterial und einem Kollektor für das trockene Abfallmaterial ausgestattet ist, die Wände der Kontaktabschnitte wassergekühlt sind, die Hohlräume der Kühlmäntel in den oberen Teilen der Wandungen, die mit einem Dampf-Gas-Medium in Berührung stehen, und in den unteren Teilen der Wandungen, die mit dem extrahierten Gemisch in Berührung stehen, voneinander getrennt sind,
das System der Gaszirkulation mit dem Hohlraum des Extraktors verbunden ist und für die Gaszufuhr ein Abzweigrohr, das mit dem Hohlraum des Kontaktabschnittes des Extraktors verbunden ist, einen Tieftemperaturkondensatorkühler und einen Ventilator umfasst,
die in der Trennkammer angeordnete Bewegungsschraube mit der Welle des Extraktors verbunden ist, und
die Trennwand zwischen der Trenn- und der Feuchtigkeitskammer mit einer zusätzlichen Öffnung im oberen Teil ausgestattet ist.

**4.** Vorrichtung nach Anspruch 3, bei der der zylindrische Körper des Extraktors geneigt ist.

**5.** Vorrichtung nach Anspruch 3 oder 4, bei dem das Abzweigrohr für die Entfernung des Extrakts aus der Trennkammer des Extraktors mit der Filtrationszentrifuge verbunden ist, die ihrerseits mit dem Verdampfer und dem Kondensator, die in Reihe geschaltet sind, verbunden ist.

**Revendications**

1. Procédé de production de chlorhydrate 1β,10β-époxy-13-diméthylamino-guaia-3(4)-én-6,12-olide lyophilisé à partir de la plante endémique *Artemisia glabella Kar. et Kir*. comprenant une extraction de résine à partir du produit initial, sa purification de substances sans importance, ladite séparation de résine en composants séparés par le procédé de chromatographie sur colonne avec la production d'arglabine technique qui est en outre soumise à une recristallisation, une amination et une hydrochloration avec la production de chlorhydrate d'aminoarglabine et en outre sa recristallisation, son séchage, sa lyophilisation et son identification dans lequel :

   ladite amination est effectuée en dissolvant l'arglabine cristalline dans un alcool avec l'ajout de diméthylamine à ladite solution jusqu'à ce que le pH de ladite solution atteigne 12,3 à 12,4, au stade précédent pour extraire le chlorhydrate d'aminoarglabine comprenant l'aminoarglabine, un alcool est distillé et du chloroforme est ajouté, le reste d'eau est éliminé, ladite solution est filtrée, ledit chloroforme est évaporé et de l'alcool est ajouté, ladite solution est soumise à un barbotage avec de l'hydrure de chlore jusqu'à ce que le pH de la solution atteigne 5,0 à 5,5, ledit alcool est évaporé et de l'éthylacétate est ajouté.

2. Procédé selon la revendication 1, dans lequel l'identification d'arglabine lyophilisée est effectuée par spectroscopie IR.

3. Dispositif pour une utilisation pour l'extraction d'arglabine à partir d'une matière naturelle selon la revendication 1 comprenant un extracteur horizontal comprenant un corps cylindrique divisé par cloisonnements avec des ouvertures dans pas moins de trois sections, une chambre de séparation et d'humidité, l'arbre est monté sur le même axe que ledit corps, des poches et des mélangeurs à pâles attachés audit arbre, et le chargeur, dans lequel

   ledit extracteur est fourni avec une section supplémentaire avec des parois chauffées pour éliminer l'extrait des déchets et un collecteur de déchets anhydres, les parois des sections de contact sont refroidies avec de l'eau, les cavités des chemises de refroidissement des parties supérieures des parois étant en contact avec un milieu gaz-vapeur et des parties inférieures des parois étant en contact avec le mélange extrait, ne sont pas ouvertes les unes aux autres,

   ledit système de circulation de gaz est connecté à ladite cavité de l'extracteur, ledit système pour l'alimentation du gaz comprend un conduit de raccordement connecté à la cavité de la section de contact de l'extracteur, du condensateur à basse température et du ventilateur,

   la vis-mère placée dans la chambre de séparation est connectée à l'arbre de l'extracteur,

   le cloisonnement entre les chambres de séparation et d'humidité est effectué avec une ouverture supplémentaire dans sa partie supérieure.

4. Dispositif selon la revendication 3, dans lequel le corps cylindrique des extracteurs est incliné.

5. Dispositif selon la revendication 3 ou la revendication 4, dans lequel le conduit de raccordement pour l'élimination de l'extrait de la chambre de séparation de l'extracteur est connecté à la centrifuge de filtration qui est connectée à l'évaporateur et au condensateur en série.

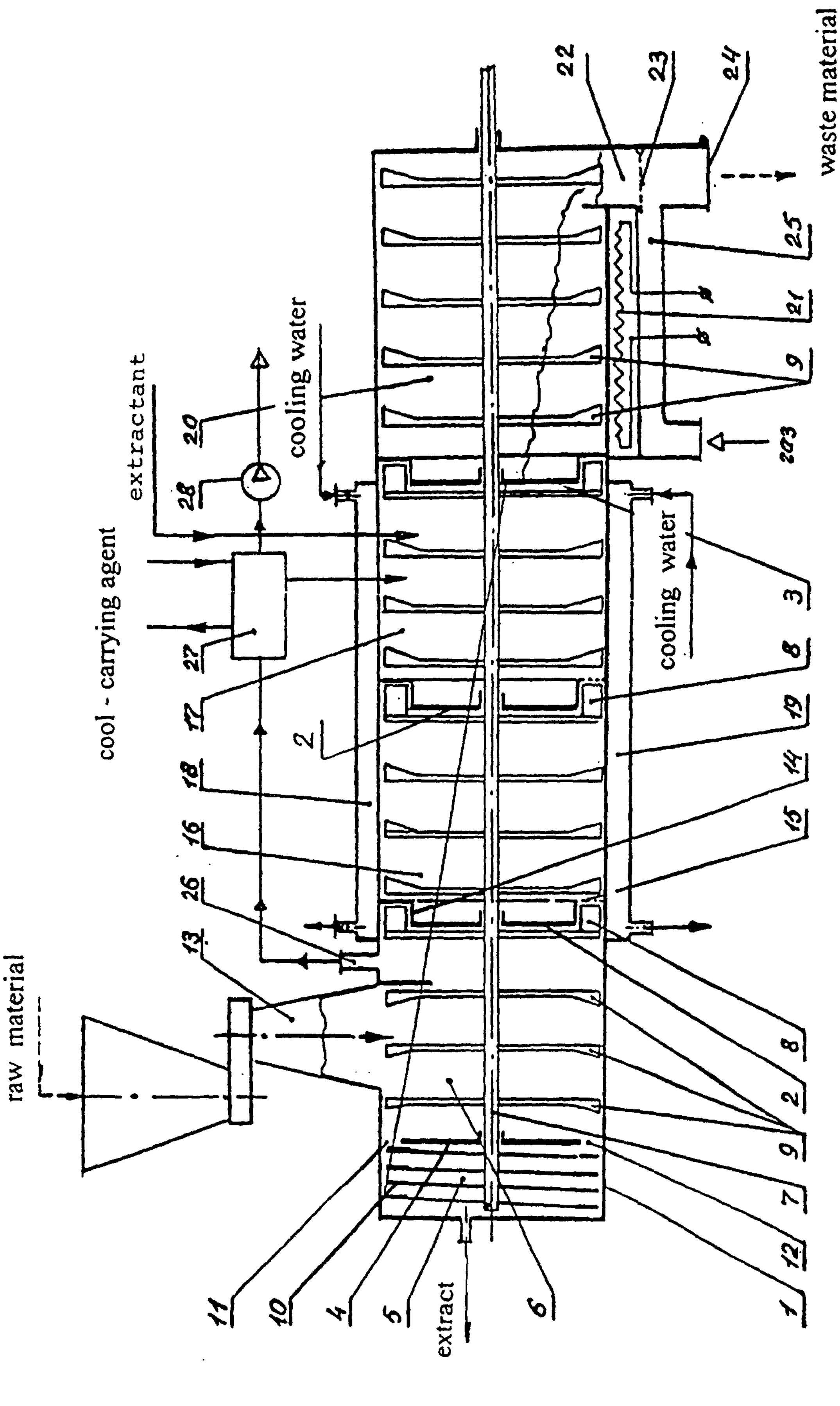
raw material
cool - carrying agent
extractant
cooling water
cooling water
waste material
extract

Fig. 1

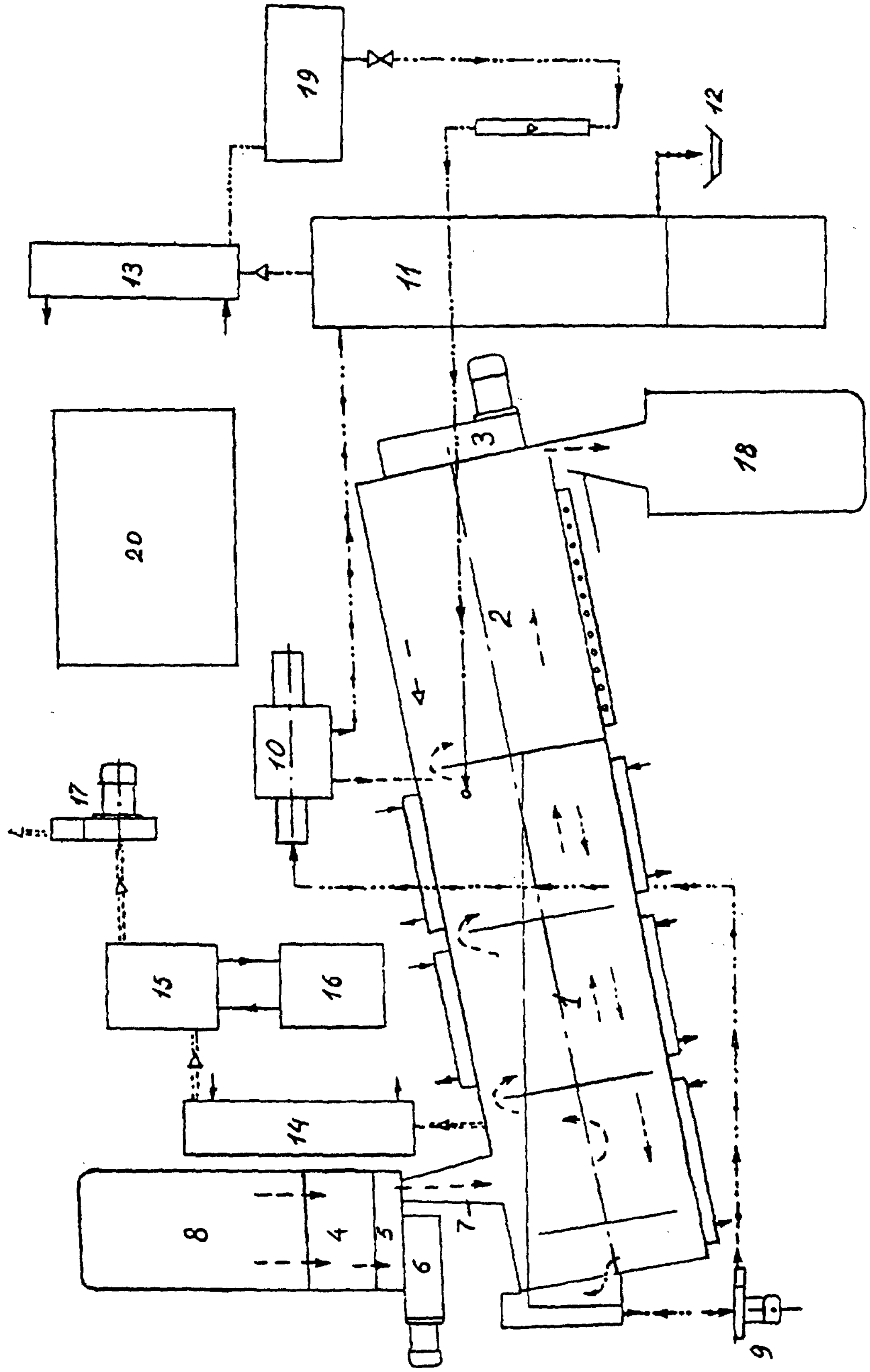


Fig. 2